Europäisches Patentamt

European Patent Office

Office européen des brevets

(11) Publication number: **0 046 017**
**B1**

(12) # EUROPEAN PATENT SPECIFICATION

(45) Date of publication of patent specification: **09.11.83**

(51) Int. Cl.³: **C 12 P 7/62**

(21) Application number: **81303246.3**

(22) Date of filing: **15.07.81**

(54) Extraction of poly(beta-hydroxy butyric acid).

(30) Priority: **13.08.80 GB 8026460**
**23.12.80 GB 8041182**

(43) Date of publication of application:
**17.02.82 Bulletin 82/7**

(45) Publication of the grant of the patent:
**09.11.83 Bulletin 83/45**

(84) Designated Contracting States:
**BE DE FR GB IT NL**

(56) References cited:
**GB - A - 1 381 306**
**US - A - 3 036 959**
**US - A - 3 044 942**

(73) Proprietor: **IMPERIAL CHEMICAL INDUSTRIES PLC**
**Imperial Chemical House Millbank**
**London SW1P 3JF (GB)**

(72) Inventor: **Walker, John**
**Clockwood House Leven Road**
**Yarm Cleveland (GB)**
Inventor: **Whitton, Jonathan Richard**
**15 Bransdale**
**Guisborough Cleveland (GB)**
Inventor: **Alderson, Barry**
**5 Sinderby Close**
**Billingham Cleveland (GB)**

(74) Representative: **Gratwick, Christopher et al,**
**Imperial Chemical Industries PLC Legal**
**Department: Patents Thames House North**
**Millbank**
**London SW1P 4QG (GB)**

Extraction of poly(beta-hydroxy butyric acid)

This invention relates to the extraction of poly($\beta$-hydoxy butyric acid) hereinafter referred to as PHB.

PHB is a thermoplastic polyester that is useful as a plastics material. It is accumulated by many bacteria as an energy reserve material in the form of granules within the bacterial cells.

While bacterial cells containing PHB can be used as such as a moulding material, for example as described in USP 3,107,172, it is generally described to separate the PHB from the remainder of the bacterial cell material.

Methods that have been proposed to effect this separation include breakage of the cells by methods such as treatment with acetone, followed by extraction of the PHB from the broken cells by treatment with a solvent in which PHB is soluble. Examples of such processes are described in USP's 3,036,959 and 3,044,942 in which the solvents employed are pyridine or mixture of methylene chloride and ethanol. Other extraction solvents for PHB in the form in which it is produced in the bacterial cells include cyclic carbonates such as 1,2-propylene carbonate (see USP 4,101,533); chloroform (see USP 3,275,610); and 1,2-dichloroethane (see European Patent Application 15123).

USP 3,275,610 discloses other methods of cell breakage viz. ultrasonic vibration, grinding, French pressing, freezing/thawing cycles and lysozyme treatment, while, as described in the aforementioned European Patent Application, spray or flash drying of the suspension of cells, as produced by culturing the microorganism in an aqueous medium on a suitable carbon and energy source, can also cause sufficient cell breakage to enable the PHB to be extracted from the cells.

One disadvantage of these processes is that it is necessary to separate the PHB-containing solution from the cell debris.. Because of the small size of the bacterial cells, and hence of the fragments resulting therefrom, techniques such as filtration have heretofore presented difficulties. This is particularly true where the PHB-containing solution is relatively viscous as in the case where chloroform is utilised as the solvent. As described in the aforesaid European Patent Application, in some cases the PHB can be extracted by a wet process wherein the aqueous bacterial cell suspension, after a suitable cell breakage step, is contacted with a suitable solvent for PHB and then the solvent and aqueous phases are separated. However, separation of the phases may be slow and incomplete.

We have found, that if the bacterial cell suspension is subjected to a flocculation step, sufficient cell breakage may occur during the treatment required to effect flocculation to permit the PHB to be extracted from the cell debris. Also, as a result of the cell flocculation, separation of the PHB-containing solution from the cell debris may be more readily accomplished.

Accordingly we provide a process for the separation of PHB from an aqueous suspension of bacterial cells containing PHB comprising causing said suspension to flocculate by reducing the pH of the suspension to a value within the range 2 to 5 by treatment with an acid, the suspension being treated with an alkali to increase its pH to a value in the range 8 to 12 before acidification and/or heated to a temperature within the range 50 to 200°C before or after acidification, separating the flocculated cells from the aqueous medium, extracting PHB from the flocculated cells by contacting the latter with a solvent in which the PHB is soluble, and separating the solvent having the PHB dissolved therein from the cell debris.

Examples of such flocculation process are described in UK Patent Specifications 1,062,005 and 1,381,306. Preferably the suspension is flocculated by increasing its pH to a value in the range 8.5 to 12, heating by injecting steam under pressure into the cell suspension, and then acidifying to a pH in the range 3 to 5.

The amount and temperature of the steam is preferably such as to raise the temperature of the cell suspension to a temperature in the range 60 to 100°C.

The flocculated cells may be separated from the aqueous medium by filtration, sedimentation, flotation, centrifugation or a drying technique such as spray drying.

Before being contacted with the PHB-extraction solvent, the flocculated cells are preferably contacted with a solvent in which the lipids associated with the bacterial cell are soluble but in which PHB is insoluble. Examples of such solvents include methanol and acetone. The extraction of lipids is preferably effected at elevated temperatures, e.g. 40 to 90°C, although sufficient lipid extraction may be effected in some cases using lower temperatures e.g. 25 to 40°C. The use of the elevated temperatures is preferred as in general, when using such elevated temperatures, the flocculated cells tend to sink in the lipid extraction solvent thus rendering separation of the flocculated cells and lipid extraction solvent facile by techniques such as decanting.

The cells are then contacted with the PHB-extraction solvent. Preferred extraction solvents include 1,2-dichloroethane, methylene chloride and chloroform. Where no preliminary lipid extraction step is employed, the PHB extraction is preferably effected at temperatures below 40°C, whereas higher temperatures, e.g. 50 to 90°C may advantageously be employed if a preliminary lipid extraction step is utilised.

The lipid extraction step (if used) and/or the PHB extraction may be effected continuously with the flocculated cells packed into a suitable bed.

# 0 046 017

In one preferred form of the invention the flocculated cells are dried, for example in a fluid bed drier, after lipid extraction. This results in a relatively porous granular material which can then be contacted with the PHB extraction solvent. We have found that with such granular materials, the PHB may readily be leached therefrom leaving the cell debris in the granular form: this granular cell debris can be separated from the PHB-containing solution easily by techniques such as filtration. The porous granular material formed by drying the lipid extracted flocculated cells is particularly suitable for trickle extraction techniques wherein the PHB-solvent is allowed to permeate down through a bed of the granular material.

In another form of the invention the flocculated cells are subjected to a lipid extraction step as described hereinbefore, separated from the lipid extraction solvent and then reslurried in water. The resultant slurry may then be subjected to a 'wet' extraction process, e.g. as described in aforesaid European Patent Application 15123, by adding a liquid that is immiscible with water and in which PHB is soluble to the slurry.

Generally no further cell breakage step, e.g. milling as was proposed for use in some cases in aforesaid European Application 15123, is necessary where the flocculated cells have been subjected to a lipid extraction step.

After agitation of the aqueous slurry with the PHB extraction solvent, the two liquid phases may be separated: the cell debris remains in the aqueous phase while the PHB is dissolved in the solvent phase. The PHB extraction solvents mentioned hereinbefore, viz. chloroform, 1,2-dichloroethane, and methylene chloride may be used to effect the PHB extraction by this process.

Because of the lipid removal step prior to the 'wet' extraction, the formation of emulsions between the two liquid phases is avoided, and separation thereof is relatively simple.

The PHB may be recovered from the solution in the extraction solvent by precipitation into a nonsolvent, e.g. a methanol/water mixture or by evaporation of the solvent, e.g. by spray or flash drying.

The invention is illustrated by the following examples:

### Example 1:

An aqueous suspension of *Alcaligenes eutrophus* of 150 g $l^{-1}$ biomass content, of which about 45% by weight was PHB, was flocculated by addition of alkali to increase the pH to 9, heated to 90°C for 10 min and then acidified to pH 5. The resultant flocs were separated from the aqueous medium by decanting.

100 ml of the wet flocs were added to 200 ml of methanol and refluxed for 5 min. The methanol was then removed by decanting and the resultant flocs dried in a fluid bed dried at 60°C for 20 min. A granular product was formed.

10 g of the granular product were refluxed with 200 ml of chloroform for 5 min to extract PHB. The cell debris was granular and floated on top of the chloroform solution and was readily skimmed therefrom.

The PHB was precipitated from the chloroform solution by adding the solution to a mixture of methanol and water (4 vol. methanol : 1 vol. water). The precipitated PHB was recovered by filtration and dried in an oven at 40°C. The amount of PHB recovered corresponded to about 80% by weight of that in the *Alcaligenes eutrophus* bacterial cells.

The weight average molecular weight of the recovered PHB was 270,000 as measured by gel permeation chromatography.

### Example 2 (comparative)

By way of comparison, PHB was extracted by refluxing 10 g of dried cells (obtained by spray drying the aqueous suspension) with 200 ml of chloroform. The cell debris was in the form of fine particles that could only be separated from the chloroform solution with difficulty.

### Example 3

Example 1 was repeated except that instead of refluxing the granules with the chloroform, the granules were mixed at room temperature with the chloroform in a Silverson mixer. The cell debris was easier to separate from the chloroform solution than in Example 2 but more difficult than in Example 1.

The amount of PHB recovered was about 50% by weight of that in the bacterial cells.

### Example 4

It has been proposed in USP 3036959 to treat wet bacterial cells with acetone prior to extraction with a PHB extraction solvent. The amount of acetone suggested is 1 to 10 times the weight of the cells.

To examine the effect of acetone, to portions of an aqueous suspension containing about 5% by weight of cells of which about 50% by weight was PHB, there were added various amounts of acetone and the mixture shaken for 2 minutes at room temperature.

3

| Sample | Acetone ml | Cell suspension ml | Result |
|--------|-----------|-------------------|--------|
| A | 10 | 90 | No visible effect |
| B | 50 | 50 | The cells assumed a partially flocculated appearance but no separation of the cells from the liquid phase occurred. |
| C | 90 | 10 | The cells assumed a partially flocculated appearance and settled to occupy a volume of about 25 ml. |

Since treatment of wet cells, or the cell suspension, with acetone would give rise to the need to recover the acetone from the acetone/aqueous medium mixture when operated on a large scale, the effect of acetone on dried cells was examined.

Various amounts of spray dried cells, as used in Example 2, or air dried cells, i.e. cells that had been separated from the suspension as used in Example 1 by centrifugation followed by fluid bed drying in air at 40°C, were agitated for 2 minutes with 100 ml of acetone at room temperature, and the suspension left to stand for 1 hour. The cells did not have a flocculated appearance but sedimented to some degree as shown in the following table.

| Sample | Type of cells | Weight of cells (g) | Amount of cells sedimented (g) |
|--------|--------------|--------------------|-------------------------------|
| D | Spray dried | 2 | trace |
| E | Spray dried | 5 | ~2 |
| F | Spray dried | 10 | ~8 |
| G | Air dried | 2 | trace |
| H | air dried | 5 | ~1 |
| I | air dried | 10 | ~5—6 |

When the sedimented cells of Samples C or F were separated by decanting, dried, and refluxed with chloroform, separation of the cell debris from the chloroform solution was no easier than in Example 2.

**Claims**

1. A process for the separation of poly($\beta$-hydroxy butyric acid), PHB, from an aqueous suspension of bacterial cells containing PHB wherein the PHB-containing cells are contacted with an extraction solvent in which PHB is soluble and the solvent having the PHB dissolved therein is separated from the cell debris, characterised in that the bacterial cells in the aqueous suspension are caused to flocculate by the step of reducing the pH of the suspension to a value in the range 2 to 5 by treatment with an acid, in combination with at least one of the steps (i) treatment of the suspension with alkali to increase its pH to a value in the range 8 to 12 before acidification, and (ii) heating the suspension to a temperature within the range 50 to 200°C before or after acidification, and then the flocculated cells are separated from the aqueous medium prior to contact of the cells with the extraction solvent.

2. A process according to claim 1 characterised in that the cells in the suspension are caused to flocculate by increasing the pH of the suspension to a value in the range 8.5 to 12, heating by injecting steam under pressure into the suspension, and then acidifying the suspension to a pH in the range 3 to 5.

3. A process according to claim 2 characterised in that the cell suspension is heated to a temperature in the range 60 to 100°C by the injection of steam.

4. A process according to any one of claims 1 to 3 characterised in that, prior to contact with the extraction solvent, the lipids associated with the bacterial cells are extracted from the flocculated cells

4

by contact with a solvent in which the lipds are soluble but in which PHB is insoluble, and the solvent having the lipids dissolved therein is separated from the flocculated cells.

5. A process according to claim 4 characterised in that the flocculated cells are contacted with the lipid extraction solvent at a temperature in the range 40 to 90°C.

6. A process according to claim 4 or claim 5 characterised in that the lipid extraction solvent is acetone or methanol.

7. A process according to any one of claims 4 to 6 characterised in that, after lipid extraction but before contact with the PHB extraction solvent, the flocculated cells are dried whereby a porous granular product is obtained for contact with the PHB extraction solvent.

8. A process according to any one of claims 4 to 6 characterised in that, after the flocculated cells are separated from the lipid extraction solvent, the flocculated cells are slurried in water prior to contact with the PHB extraction solvent.

9. A process according to any one of claims 1 to 8 characterised in that the PHB extraction solvent is selected from chloroform, 1,2-dichloroethane, and methylene chloride.

**Patentansprüche**

1. Verfahren zur Abtrennung von Poly-$\beta$-hydroxybuttersäure (PHB) von einer wäßrigen Suspension PHB enthaltender Bakterienzellen, bei dem die PHB enthaltenden Zellen mit einem Extraktionslösungsmittel, in dem PHB löslich ist, in Berührung gebracht werden und das Lösungsmittel mit der darin gelösten PHB von den Zellresten abgetrennt wird, dadurch gekennzeichnet, daß die in der wäßrigen Suspension befindlichen Bakterienzellen durch den Schritt der Verminderung des pH der Suspension auf einen im Bereich von 2 bis 5 liegenden Wert durch Behandlung mit einer Säure in Verbindung mit mindestens einem der folgenden Schritte:

(i) Behandlung der Suspension mit Alkali zur Erhöhung ihres pH auf einen im Bereich von 8 bis 12 liegenden Wert vor dem Ansäuren und

(ii) Erhitzen der Suspension auf eine innerhalb des Bereichs von 50 bis 200°C liegende Temperatur vor oder nach den Ansäuern

ausgeflockt werden und daß die ausgeflockten Zellen dann vor ihrer Berührung mit dem Extraktionslösungsmittel von dem wäßrigen Medium abgetrennt werden.

2. Verfahren nach Anspruch 1, dadurch gekennzeichnet, daß die in der Suspension befindlichen Zellen dadurch ausgeflockt werden, daß der pH der Suspension auf einen im Bereich von 8,5 bis 12 liegenden Wert erhöht wird, daß durch Einblasen von Dampf unter Druck in die Suspension erhitzt wird und daß die Suspension dann bis zu einem im Bereich von 3 bis 5 liegenden pH-Wert angesäuert wird.

3. Verfahren nach Anspruch 2, dadurch gekennzeichnet, daß die Zellsuspension durch Einblasen von Dampf auf eine Temperatur im Bereich von 60 bis 100°C erhitzt wird.

4. Verfahren nach einem der Ansprüche 1 bis 3, dadurch gekennzeichnet, daß die mit den Bakterienzellen assoziierten Lipide vor der Berührung mit dem Extraktionslösungsmittel durch Berührung mit einem Lösungsmittel, in dem die Lipide löslich sind, in dem jedoch die PHB unlöslich ist, aus den ausgeflockten Zellen extrahiert werden und daß das Lösungsmittel mit den darin gelösten Lipiden von den ausgeflockten Zellen abgetrennt wird.

5. Verfahren nach Anspruch 4, dadurch gekennzeichnet, daß die ausgeflockten Zellen mit dem Lipidextraktionslösungsmittel bei einer Temperatur im Bereich von 40 bis 90°C in Berührung gebracht werden.

6. Verfahren nach Anspruch 4 oder 5, dadurch gekennzeichnet, daß das Lipidextraktionslösungsmittel Aceton oder Methanol ist.

7. Verfahren nach einem der Ansprüche 4 bis 6, dadurch gekennzeichnet, daß die ausgeflockten Zellen nach der Lipidextraktion, jedoch vor der Berührung mit dem PHB-Extraktionslösungsmittel, getrocknet werden, wodurch ein poröses, körniges Produkt für die Berührung mit dem PHB-Extraktionslösungsmittel erhalten wird.

8. Verfahren nach einem der Ansprüche 4 bis 6, dadurch gekennzeichnet, daß die ausgeflockten Zellen nach ihrer Abtrennung von dem Lipidextraktionslösungsmittel und vor der Berührung mit dem PHB-Extraktionslösungsmittel in Wasser aufgeschlämmt werden.

9. Verfahren nach einem der Ansprüche 1 bis 8, dadurch gekennzeichnet, daß das PHB-Extraktionslösungsmittel aus Chloroform, 1,2-Dichloroethan und Methylenchlorid ausgewählt ist.

**Revendications**

1. Procédé pour séparer l'acide poly($\beta$-hydroxybutyrique), PHB, d'une suspension aqueuse de cellules bactériennes contenant du PHB, suivant lequel on met les cellules contenant du PHB en contact avec un solvant d'extraction dans lequel le PHB est soluble et on sépare des débris cellulaires le solvant contenant le PHB dissous, caractérisé en ce qu'on fait floculer les cellules bactériennes de la suspension aqueuse en abaissant le pH de la suspension jusqu'à une valeur de l'intervalle de 2 à 5 par traitement au moyen d'un acide, en combinaison avec au moins l'un des stades (i) de traitement de la suspension au moyen d'un alcali afin d'élever son pH jusqu'à une valeur de l'intervalle de 8 à 12 avant l'acification et (ii) de chauffage de la suspension jusqu'à une température de l'intervalle de 50 à 200°C avant ou après l'acidification, puis on sépare les cellules floculées du milieu aqueux avant de mettre les cellules en contact avec le solvant d'extraction.

2. Procédé suivant la revendication 1, caractérisé en ce qu'on fait floculer les cellules de la suspension par élévation du pH de la suspension jusqu'à une valeur de l'intervalle de 8,5 à 12, par chauffage au moyen d'une injection de vapeur d'eau sous pression dans la suspension et ensuite par acidification de la suspension jusqu'à un pH de l'intervalle de 3 à 5.

3. Procédé suivant la revendication 2, caractérisé en ce qu'on chauffe la suspension de cellules jusqu'à une température de l'intervalle de 60 à 100°C par l'injection de vapeur d'eau.

4. Procédé suivant l'une quelconque des revendications 1 à 3, caractérisé en ce qu'avant le contact avec le solvant d'extraction, on extrait les lipides associés aux cellules bactériennes hors des cellules floculées par contact avec un solvant dans lequel les lipides sont solubles, mais le PHB est insoluble et on sépare le solvant contenant les lipides dissous des cellules floculées.

5. Procédé suivant la revendication 4, caractérisé en ce qu'on met les cellules floculées en contact avec le solvant pour l'extraction des lipides à une température de l'intervalle de 40 à 90°C.

6. Procédé suivant la revendication 4 ou 5, caractérisé en ce que le solvant pour l'extraction des lipides est l'acétone ou le méthanol.

7. Procédé suivant l'une quelconque des revendications 4 à 6, caractérisé en ce qu'après l'extraction des lipides, mais avant le contact avec le solvant pour l'extraction du PHB, on sèche les cellules floculées de manière à obtenir un produit granulaire poreux en vue du contact avec le solvant pour l'extraction du PHB.

8. Procédé suivant l'une quelconque des revendications 4 à 6, caractérisé en ce qu'après que les cellules floculées ont été séparées du solvant pour l'extraction des lipides, on disperse les cellules floculées dans de l'eau avant le contact avec le solvant pour l'extraction du PHB.

9. Procédé suivant l'une quelconque des revendications 1 à 8, caractérisé en ce que le solvant pour l'extraction du PHB est choisi entre le chloroforme, le 1,2-dichloroéthane et le chlorure de méthylène.